(19) Europäisches Patentamt
European Patent Office
Office européen des brevets

(11) **EP 3 351 162 A1**

(12) **EUROPEAN PATENT APPLICATION**

(43) Date of publication:
**25.07.2018 Bulletin 2018/30**

(51) Int Cl.:
***A61B 5/00*** *(2006.01)*

(21) Application number: **17152349.1**

(22) Date of filing: **20.01.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Universitat Politècnica De Catalunya 08034 Barcelona (ES)**

(72) Inventors:
- **DELPUEYO, Xana**
  **08784 Piera (ES)**
- **VILASECA, Meritxell**
  **08222 Terrassa (ES)**
- **ARES, Miguel**
  **08005 Barcelona (ES)**
- **ROYO, Santiago**
  **08755 Castellbisbal (ES)**
- **VÁZQUEZ, Jorge**
  **08024 Barcelona (ES)**

- **PELLACANI, Giovanni**
  **41124 Modena (IT)**
- **ANDRE, Philipp**
  **70199 Stuttgart (DE)**
- **SOLOMITA, Giuseppe**
  **80469 München (DE)**
- **BASSOLI, Sara**
  **41126 Modena (IT)**
- **MALVEHY, Josep**
  **08810 Sant Pere de Ribes (ES)**
- **PUIG, Susana**
  **08810 Sant Pere de Ribes (ES)**
- **PERCHOUX, Julien**
  **31500 Toulouse (FR)**
- **QUOTB, Adam**
  **81600 Gaillac (FR)**

(74) Representative: **Juncosa Miro, Jaime et al Torner, Juncosa i Associats, S.L. Gran Via de les Corts Catalanes, 669 bis, 1o, 2a 08013 Barcelona (ES)**

(54) **A COMPUTER IMPLEMENTED METHOD, A SYSTEM AND COMPUTER PROGRAM PRODUCTS TO CHARACTERIZE A SKIN LESION**

(57) The method comprises storing three sets of images acquired by three different optical technologies including multispectral (101), 3D (102) and optical feedback interferometry, OFI, (103); analyzing two of said three sets computing a series of indicators and/or parameters for each one of said two analyzed sets of images; and combining the two series of computed indicators and/or parameters to characterize the skin lesion. The set of images acquired by the multispectral technology (101) are acquired using different illumination spectral bands to detect color and spectral properties of the skin lesion and their spatial distribution, the set of images acquired by the 3D technology (102) are acquired using stereoscopy and/or fringe projection techniques to detect three-dimensional morphological properties of the skin lesion and their spatial distribution, and the set of images acquired by the OFI technology (103) are acquired to detect blood flow changes of the skin lesion and their spatial distribution.

Fig. 2

**Description**

Technical Field

The present invention is directed, in general, to the field of medical imaging methods and systems for treating diseases. In particular, the invention relates to a computer implemented method, a system and computer program products to characterize a skin lesion such as a skin cancer lesion or a benign lesion.

Background of the Invention

The incidence of skin cancer is rising rapidly. A person has a 1:33 chance to develop melanoma, the most aggressive type of skin cancer. It is the second most common cancer in young adults (aged 15-34) and twice as common in women in this age group. About 90% of skin cancers are caused by ultraviolet (UV) light in sunlight. Skin cancer accounts for one in three cancers worldwide. The 5 year survival rate for people with skin cancers significantly improves if detected and treated early. A significant improvement of the current diagnostic tools of dermatologists is required in order to identify dermal disorders at a very early stage as well as to monitor directly the effects of treatment. Its early diagnosis is thus essential since it can be treated more effectively on early detection, improving the prognosis of the disease.

Nowadays, visual inspection through the dermoscope is the technique most widely used by dermatologists for the detection of skin cancer. It consists of a handheld device with a magnifying lens and a white and uniform illumination field. The light is usually polarized to remove specular reflection from the skin surface to obtain information of deeper tissue layers. Dermoscopy allows the specialists to identify different structures, patterns and colors of the skin lesions suggesting if they are benign (seborrheic keratosis, hemangioma, lipoma, wart, etc.) or malignant (melanoma, basal cell carcinoma, etc.). This is confirmed by an ulterior histological examination which requires a surgical procedure involving the extraction of the lesion, which is currently the gold standard for clinicians. By means of this procedure, a lot of false positives are still usually obtained, and thus the direct annual costs of diagnosis and treatment of skin cancer are high.

In order to improve the early detection and diagnosis of skin cancer, different biophotonic techniques are available to provide noninvasive information due to the penetration depth of light into the first layers of skin, where skin lesions evolve. Color and spectral imaging technologies have started to be used recently to enhance and analyze color and spectral properties of skin at the surface and in the superficial surface layers. The changes in those properties are caused by chromophores such as melanin, hemoglobin, water etc., which might differ among skin lesions of different etiologies.

In this context, some prototypes such as those developed by Bekina et al. [1], Kapsokalivas et al. [2], and also some commercial devices such as the SIAscope V [3] or MelaFind systems [4], have already been proposed as a means of improving skin cancer diagnosis. However, some of them only use three spectral bands in the visible range (typically three color RGB channels), which limits their spectral resolution, and additionally, while other devices add on some additional channel in the near infrared range. Moreover, many of them only analyze the averaged color and spectral properties over the lesion.

Besides, some patents or patents applications are known in the field. For instance, patent US-B1-6208749 proposes a system to detect melanoma using a multispectral technology. Patent EP-B1-1002253 proposes a handheld confocal imaging system for in vivo observation of dermal and subdermal tissue allowing diagnosis of conditions substantially beneath the surface of the skin. Patent AU-B2-2009204227 proposes an imaging system to detect skin cancer lesions creating a unique spectral fingerprint based on the convolution of RGB color channel spectral plots generated from digital images that have captured single and/or multi-wavelength light-matter interaction. Patent application US-A1-2012123275 proposes a process for identifying and classifying cutaneous melanoma. A FTIR micro-imaging is used for characterizing various melanoma types to enable the correlation between IR spectral markers in primary melanoma and some dermatopathological parameters recognized as powerful prognostic factors. Finally, patent US-B2-8543519 proposes a system and method for photographing and downloading images of potential melanoma lesions for processing using learning machines to provide a preliminary risk assessment of melanoma.

It is desirable to provide, therefore, new imaging methods and systems to characterize a skin lesion that combine several different imaging technologies that enables fast and reliable detection of malignant and benign skin tissue with earlier, faster and more objective skin cancer screening procedures when compared to the known approaches.

References:

[1] Bekina, A., Diebele, I., Rubins, U., Zaharans, J., Derjabo, A., Spigulis, J., 2012. Multispectral assessment of skin malformations using a modified video-microscope, Latvian Journal of Physics and Technical Sciences 49(5), 4-8.

[2] Kapsokalyvas, D., Bruscino, N., Alfieri, D., de Giorgi, V., Cannarozzo, G., Cicchi, R., Massi, D., Pimpinelli, N., Pavone, F.S., 2013. Spectral morphological analysis of skin lesions with a polarization multispectral dermoscope. Optics express 21(4), 4826-40.

[3] Emery J.D., Hunter, J., Hall, P.N., Watson, A.J., Moncrieff, M., Walter, F.M., 2010. Accuracy of SIAscopy for pigmented skin lesions encountered in primary care: development and validation of a new diagnostic algorithm, BMC Dermatology, 10(9), 1:9.

[4] Monheit G., Cognetta A., Ferris L., et al., 2011. The performance of MelaFind: a prospective multicenter study, Archives of dermatology, 147(2), 188:94.

[5] Diebele I., Kuzmina I., Lihachev A., Kapostinsh J., Derjabo A., Valeine L, Spigulis J., 2012, Clinical evaluation of melanomas and common nevy by spectral imaging. Biomedical Optics Express, 3(3), 467-472.

[6] J. Emery et al, "Accuracy of SIAscopy for pigmented skin lesions encountered in primary care: development and validation of a new diagnostic algorithm", BMC Dermatology. 10, 9 (2010), [doi: 10.1186/1471-5945-10-9].

Description of the Invention

To that end, the present invention provides, according to one aspect, a computer implemented method to characterize a skin lesion, the method comprising storing in a memory three different sets of images acquired by an imaging platform comprising three different optical technologies including a multispectral technology, a 3D technology and an optical feedback interferometry (OFI) technology; analyzing at least two of the three sets of images computing a series of indicators and/or parameters for each one of said at least two analyzed sets of images; and combining the at least two series of computed indicators and/or parameters to characterize the skin lesion.

According to the proposed method, each set of images is acquired by one of said optical technologies. Preferably, this is done by illuminating a region of the skin relating to a skin lesion and by capturing reflected light therefrom. In addition, each set of images includes one or more images of the skin lesion.

Moreover, the set of images acquired by the multispectral technology (or first set of images) is acquired by using different illumination spectral bands, each illumination spectral band used providing an image, to detect color and spectral properties of the skin lesion and their spatial distribution. The set of images acquired by the 3D technology (or second set of images) is acquired by using stereoscopy and/or fringe projection techniques to detect three-dimensional morphological properties of the skin lesion and their spatial distribution. Finally, the set of images acquired by the OFI technology (or third set of images) is acquired to detect blood flow changes of the skin lesion and their spatial distribution.

In an embodiment, the series of indicators and/or parameters for the first set of images are computed by comparing, pixel by pixel, the first set of images of the skin lesion and a reference image, computing from the first set of images different radiometric magnitudes, providing a reflectance or absorbance image; computing from said reflectance or absorbance image other images whose intensity pixels represent parameters based on color coordinates or color differences of any standard color representation space, such as those of the CIELAB color space, or simply based on other empirically obtained by operating with reflectance/absorbance values of different wavelengths to enhance any particular spectral feature that can be different in healthy and malignant tissue, and compute from said other images statistical properties thereof including the computing of a histogram of the intensity of the pixels of said other images; and computing different parameters including central moment, energy, entropy and other first or second order statistical descriptors of the computed histogram. Plus, some other average, maximum, minimum or standard deviation parameters related to absorbance, reflectance, or color measurements in different spectral bands are also preferably computed.

In addition, the series of indicators and/or parameters for the second set of images are computed by combining at least six images of the second set of images of the skin lesion extracting a 3D topography image; computing from the extracted 3D topography image statistical properties thereof including the computing of a histogram of a height distribution of the 3D topography image, a histogram of a curvature distribution of the 3D topography image and a histogram of the roughness values of the 3D topography image; and computing different parameters including central moment, energy, entropy and other first or second order statistical descriptors of the computed histograms. Plus some other average, maximum, minimum, or standard deviation values related to surface topography, including perimeter, area, height, shape, roughness, volume or color measurements are also preferably computed.

Moreover, the series of indicators and/or parameters for the third set of images are computed by extracting a false color image from the third set of images related to local blood flow distribution; computing from the false color image statistical properties thereof including the computing of a histogram of the intensity of the pixels of the false color image representing blood flow; and computing different parameters including area, shape, central moment, energy, entropy and skewness of the computed histogram. Plus some other average, maximum, minimum, or standard deviation values related to measured blood flow in the region of the lesion are also preferably computed.

In an embodiment, the combining step comprises comparing the at least two series of computed indicators and/or parameters, providing a combined set of indicators and/or parameters, and further performing a statistical analysis over the combined set of indicators and/or parameters to consider only those indicators and/or parameters of the combined set having a value over a given threshold.

Preferably, the different illumination spectral bands include at least eight spectral bands, comprised in the visible and/or in the infrared domain. In an embodiment, the at least eight multispectral images are obtained through an automated and sequential process.

In yet another embodiment, the imaging platform further comprises a reflectance confocal microscopy optical technology. So, in this case, the proposed method further stores a fourth set of images acquired by the reflectance confocal microscopy optical technology to form an image of a region of the skin located beneath the skin lesion. Preferably, this is done using a laser beam in the visible or near infrared range. This set of images can then be also considered for said analysis and combination steps.

Embodiments of the present invention also provide, according to another aspect, a system to characterize a skin lesion. The system comprises one or more memories adapted to store at least three different sets of images, said at least three different sets of images being acquired by an imaging platform comprising three different optical technologies including a multispectral technology, a 3D technology and an optical feedback interferometry (OFI) technology; and one or more processors being adapted and configured to execute an algorithm to analyze at least two of said three sets of images computing a series of indicators and/or parameters for each one of said at least two analyzed sets of images; and to combine the at least two series of computed indicators and/or parameters to characterize the skin lesion.

Other embodiments of the invention that are disclosed herein include software programs to perform the method embodiment steps and operations summarized above and disclosed in detail below. More particularly, a computer program product is one embodiment that has a computer-readable medium including computer program instructions encoded thereon that when executed on at least one processor in a computer system causes the processor to perform the operations indicated herein as embodiments of the invention.

Present invention, by analyzing different sets of images obtained by several imaging technologies like 3D imaging, multispectral, OFI, and also reflectance confocal microscopy imaging provides substantial advances to skin lesion detection at earlier stages in comparison to current approaches.

Moreover, present invention, enables fast and reliable detection of malignant and benign tissue lesions without destruction of the organic material in the sample.

Brief Description of the Drawings

The previous and other advantages and features will be more fully understood from the following detailed description of embodiments, with reference to the attached figures, which must be considered in an illustrative and non-limiting manner, in which:

Fig. 1 illustrates in a simplified way the architecture of the present invention to characterize a skin lesion, according to an embodiment of the present invention.

Figs 2 and 3 depict flow charts of a method to characterize a skin lesion according to different embodiments of present invention.

Detailed Description of Preferred Embodiments

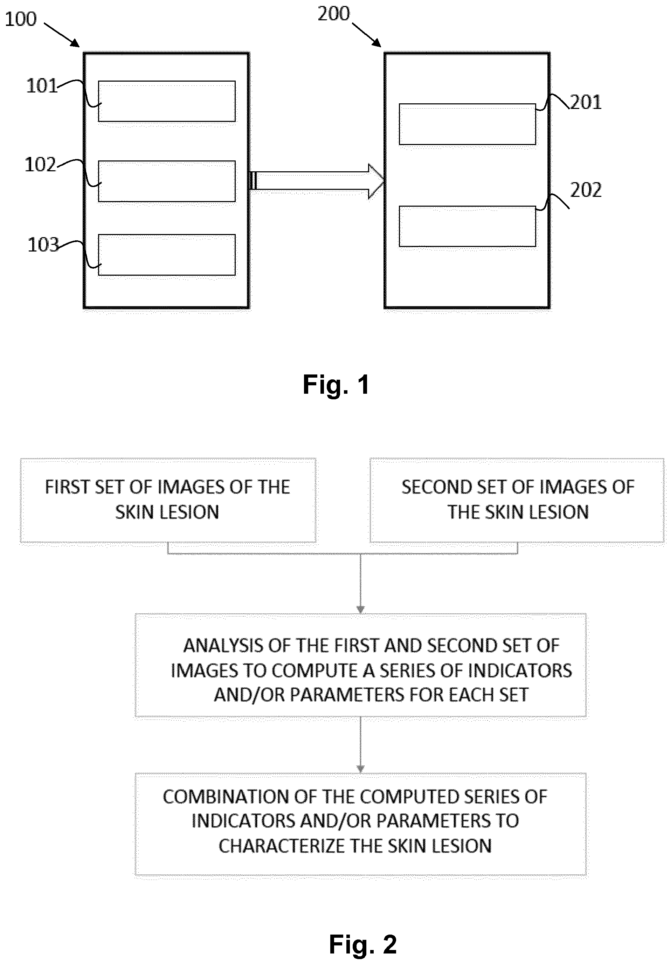

Fig. 1 shows an embodiment of the architecture of the present invention to characterize a skin lesion, i.e. to confirm if a skin lesion is malignant or benign. According to this embodiment, an imaging platform 100 which includes different optical technologies, particularly a multispectral measurement unit 101, a three-dimensional (3D) measurement unit 102 and an optical feedback interferometry (OFI) measurement unit 103 is configured to acquire different sets of images, one set for each optical technology of the imaging platform 100. Preferably, the three different optical technologies are arranged to illuminate a region of the skin relating to a skin lesion and to capture reflected light therefrom.

It should be noted that each set of images may comprise one or more images of the skin lesion.

The set of images acquired by the multispectral technology 101 (from now on referred as first set of images) is acquired by using different illumination spectral bands (at least 8 as will be shown next). Each illumination spectral band used provides an image to detect color and spectral properties of the skin lesion and their spatial distribution. Multispectral imaging is a non-invasive technology capable of providing spectroscopic data from a given image. Meanwhile in conventional spectroscopy data is obtained for each wavelength within a spectral range but only for a single spot, which is supposed to be uniform, multispectral imaging combines spectroscopic elements with a camera, allowing the recording of spectral data for each pixel of the image. A spectrograph located in front of the camera can be used to diffract the light into the wavelengths although often narrowband interference or tunable filters are used instead to generate the spectral bands. However, light sources with narrow band emission, such as light emitting diodes, can also be used nowadays.

The set of images acquired by the 3D technology 102 (referred as second set of images) is acquired by using stereoscopy and/or fringe projection techniques to detect three-dimensional morphological properties of the skin lesion and their spatial distribution. Stereoscopy using structured light projection is an optical technology that provides accurate 3D measurements of the surface of human skin. The technique is based on the projection of a set of fringes onto the skin surface. These fringes are distorted due to the skin shape and acquired by two cameras. The distortion of the fringes allows for the accurate retrieval of the 3D geometric points of the skin surface for instance by triangulation. The technology is human-safe because uses non-ionizing light to interact with the skin, and measures in-vivo the 3D topography of skin yielding a faster and more accurate skin surface characterization. The acquisition of skin images to reconstruct the surface in 3D is very fast (around one second), overcoming the involuntary in-vivo movements of a person. The technology can resolve surface changes in height up to the micron size in skin areas of some centimeters, becoming a great tool to accurately analyze the skin surface of a melanoma affected by a specific degree of ulceration, which is a breakdown of the skin over the melanoma.

The set of images acquired by the OFI technology 103 (referred as third set of images) is acquired to detect blood flow changes of the skin lesion and their spatial distribution. OFI is a sensing technique where light scattered at the diffusive object under test, in this case the skin surface, interferes inside the laser cavity modifying the emission properties of the laser. The laser is usually a laser diode, although other embodiments are possible. Such a simple setup with the laser pointing to the skin enables to measure changes in the optical path between the laser and the target, or measurements of the velocity of the skin using the Doppler effect, which is also used to measure flow. By using some scanning strategy (e.g. a micromirror device, not limitative as other scanning devices can be used) this pointwise measurement generates a map of the changes in the optical path or a false color image with the distribution of blood flow.

Referring back to Fig. 1, next, the different sets of images acquired are stored in a memory 201 (it could be more than one) of a computing system 200, to be further analyzed by one or more processors 202 of the computing system 200. The one or more processors 202 executes an algorithm (not illustrated) to analyze the acquired sets of images computing a series of indicators and/or parameters and combining the computed series of indicators and/or parameters to characterize the skin lesion. In an embodiment, said combination is performed by comparing the series of computed indicators and/or parameters, providing a combined set of indicators and/or parameters, and further performing a statistical analysis over the combined set of indicators and/or parameters to consider only those indicators and/or parameters of the combined set having a value over a given threshold (that is, only the most relevant values are considered to characterize the skin lesion).

To characterize the skin lesion, the algorithm may analyze only two of the three sets of images, e.g. only the first set of images and the second set of images, or alternatively may analyze the three sets of images.

Following, it will be detailed how each one of the different set of images, first, second, and third, is analyzed to compute the series of indicators and/or parameters.

Multispectral images or first set of images:

Multispectral images for the 8 wavelengths (or spectral bands) available (it could be more than eight) are obtained through, preferably, an automated and sequential process of acquisition. Afterwards, 8 reflectance images are computed by taking into account the spectral images of a reference image and also dark field images (images without spectral illumination), taken without ambient light and any skin sample placed in front of the imaging platform 100; the reflectance at each pixel $(i, j)$ for a given wavelength $\lambda_i$ is calculated as follows:

$$Refl_{\lambda i}(i,j) = Refl_{ref_{\lambda i}} \cdot \frac{DL_{\lambda i}(i,j) - DL_{0_{\lambda i}}(i,j)}{DL_{p_{\lambda i}}(i,j) - DL_{0_{\lambda i}}(i,j)} \quad (1)$$

where $Refl_{\lambda_i}(i,j)$ is the reflectance, $DL_{\lambda_i}(i,j)$ is the digital level of the raw acquired image of the skin lesion, $DL_{0_{\lambda i}}(i,j)$ is the digital level of the dark current image, $DL_p(i,j)$ is the digital level of the reference image, and $Refl_{ref_{\lambda i}}$ is the calibrated reflectance of the reference provided by the manufacturer. All images are acquired with the camera parameters of offset, gain and exposure time as those used for the analyzed skin lesion.

From the reflectance images of the skin lesion, additional images containing other parameters are computed to look for color features of skin lesions; a first group of parameters based on color coordinates of any standard color representation spaces are used, such as those of the CIELAB color space in which color is represented with lightness (L*), red-green (a*), and yellow-blue coordinates (b*); chroma ($C^*_{ab}$) and the hue angle can also be used alternatively ($h_{ab}$). In this group other more complex parameters based on color differences ($\Delta E$) between each pixel of the lesion and the averaged color of the whole lesion may be also considered as well as spectral and color differences between each pixel of the skin lesion and the averaged color of the surrounding healthy skin.

A second group of parameters that may be computed consists of the self-called empirical parameters; they are basically computed empirically by operating with reflectance values at different wavelengths trying to enhance any particular spectral feature that can be different in healthy and malignant tissue, and that can be useful to discriminate among different types of skin lesions:

$$Par_{\lambda i}(i,j) = f\big(Refl_{\lambda i}(i,j)\big) \ (2)$$

where $Par_{\lambda i}(i,j)$ is a particular parameter and $f(Refl_{\lambda i}(i,j))$ is any function of the reflectance images from different wavelengths (they can be added, subtracted, multiplied etc.) in order to highlight subtle differences among skin lesions of different etiology. Some of these parameters can be useful to resemble a map of a particular skin chromophore [3]. In this context, Diebele et al. [5] proposed the following parameters to account for bilirubin (B) or erythema (E):

$$B = I_{450}/I_{660} \ (3)$$

$$E = I_{660}/I_{545} \ (4)$$

where $I_\lambda$ is the intensity of diffuse light reflected from the skin at a specific wavelength of a multispectral system composed if 4 different spectral bands, three in the visible range (450, 545 and 660 nm) and one in the infrared (940 nm). The same authors also proposed a melanoma index (p) as follows:

$$p = k \cdot \frac{I_{545}}{I_{660} * I_{940}} \ (5)$$

where k is the intensity coefficient that describes the white etalon reference used for the calibration of the system.

Similarly, Emery et. al. [6] suggested that the SIAscope system could have an important role improving the management of pigmented skin lesions due to the fact that the system maps the dermal melanin of lesions and that it is expected that melanomas have more dermal melanin than others. Specifically, the system also captured 4 different image data (the RGB channels and one in the infrared) and to avoid the calibration of the system, the ratio images of green over red and blue over red images were calculated pixel by pixel to account for the concentrations of blood and melanin, respectively, helped with a conversion table.

Once all parameter images from the proposed system are obtained, including both color-based and empirical ones, a mask is created in order to segment the lesion from the skin with a threshold method based on the intensity of the histogram of the bluest reflectance image. In the cases that this is not possible due to the fact that the reflectance values are very low at this wavelength, the reflectance image corresponding to others available are used instead.

Then, statistical descriptors for every segmented lesion are obtained such as the mean, standard deviation, maximum and minimum, in terms of all parameters. As a first approach to the extraction of textural information, present invention also uses the analysis of the statistical properties of the histogram in terms of any of the parameters calculated. This analysis includes the study of some features such as the entropy (Ep), a statistical measure of randomness, the energy (En), a numerical descriptor of the image uniformity, and the third central moment ($\mu$3), which accounts for the skewness of the histogram. The mathematical description of these features can be seen in the following equations:

$$Ep = -\sum_{i=0}^{n-1} P_i log_2(P_i) \ (3)$$

$$En = \sum_{i=0}^{n-1} P_i{}^2 \quad (4)$$

$$\mu_3 = - \sum_{i=0}^{n-1} (i-m)^3\, P_i \quad (5)$$

where n is the number of bins or intervals in which the histogram is divided into, $P_i$ is the relative frequency of the bin $i$ of the histogram and m is the mean of the parameter.

3D images or second set of images:

At least six images of the second set of images of the skin lesion are combined extracting therefrom a 3D topography image. Then, from the extracted 3D topography image statistical properties are computed, for instance average, maximum, minimum or standard deviation of values such as the volume, shape, perimeter, slope or height of a lesion, or a histogram of a distribution of such parameters, a curvature distribution or a roughness distribution of the 3D topography image. Finally, different parameters are computed including central moment, energy, entropy and other first or second order statistical descriptors of the computed histograms, following comparable expressions to the ones just discussed above for the first set of images.

OFI images or third set of images:

A false color image presenting a distribution of blood flow values at, or around the lesion is extracted from the third set of images by scanning the laser beam in the area of the lesion.
Then, statistical properties of the false color image are computed, for instance average, maximum, minimum or standard deviation values of parameters such as blood flow, and a histogram of the intensity of the pixels of the false color image which represents now the amount of blood flow. Finally, different parameters are computed including area, shape, central moment, energy, entropy and skewness of the computed histogram, following comparable expressions to the ones just discussed above for the first and second set of images.

With reference now to Fig. 2, therein it is illustrated an embodiment of a method for characterizing a skin lesion. According to this particular embodiment, the method, once the three different sets of images acquired by the imaging platform 100 are stored in the memory 201, analyzes, via said algorithm running in the one or more processors 202, the first and second set of images to compute the series of indicators and/or parameters of each set of images, i.e. in this case only the set of images acquired by the multispectral technology 101 and the set of images acquired by the 3D technology 102 are considered. Finally, when the two series of indicators and/or parameters are computed, the same are combined to characterize the skin lesion.

Other combinations, in this case not illustrated, are also possible. For instance, the joint analysis of the first set of images and the third set of images to compute the series of indicators and/or parameters of each of this set of images used and the latter combination of them.

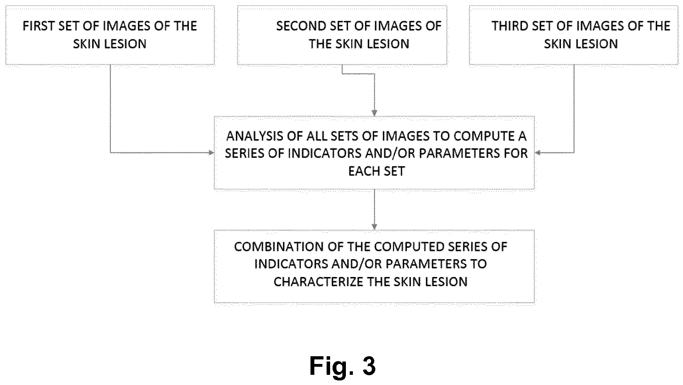

With reference to Fig. 3, therein it is illustrated another embodiment of a method for characterizing a skin lesion. In this case, different to the embodiment of Fig. 2, the three different sets of images stored in the memory are analyzed to characterize the skin lesion.

The skin lesions suspicious to be malignant, according to an embodiment, may be further analyzed using a reflectance confocal microscopy optical technology (RCM). RCM is a modern medical imaging technology that serves as an additional non-invasive tool for the diagnosis of equivocal skin lesions including screening and diagnosis, image-guided biopsy, pre- and intraoperative mapping of tumour margins to guide surgery, and monitoring of treatment efficacy. The microscope includes a laser-light source (830 nm wavelength). In the grayscale images, melanin and keratin appear bright while fluids and nuclei appear dark. The contrast in the images is dependent on differences in the refractive indices of the native components of the tissue. The image resolution of the skin layers in the horizontal plane is of a quasi-histological quality. Due to the high resolution (lateral resolution 0.5 to 1 $\mu$m, axial resolution of 3 to 5 $\mu$m corresponding to the section thickness of normal histological preparations) cellular size and shape, epidermal structure and dermal collagen morphology can be analysed.

The proposed invention may be implemented in hardware, software, firmware, or any combination thereof. If implemented in software, the functions may be stored on or encoded as one or more instructions or code on a computer-readable medium.

Computer-readable media includes computer storage media. Storage media may be any available media that can be accessed by a computer. By way of example, and not limitation, such computer-readable media can comprise RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Disk and disc, as used herein, includes compact disc (CD), laser disc, optical disc, digital versatile disc (DVD), floppy disk and Blu-ray disc where disks usually reproduce data magnetically, while discs reproduce data optically with lasers. Combinations of the above should also be included within the scope of computer-readable media. Any processor and the storage medium may reside in an ASIC. The ASIC may reside in a user device. In the alternative, the processor and the storage medium may reside as discrete components in a user device.

As used herein, computer program products comprising computer-readable media including all forms of computer-readable medium except, to the extent that such media is deemed to be non-statutory, transitory propagating signals.

The scope of the present invention is defined in the following set of claims.

## Claims

1. A computer implemented method to characterize a skin lesion, the method comprising:

   - storing in a memory (201) three different sets of images acquired by an imaging platform (100) which comprises three different optical technologies including a multispectral technology (101), a 3D technology (102) and an optical feedback interferometry, OFI, technology (103);

      wherein each set of images being acquired by one of said optical technologies (101, 102, 103), each set of images comprising one or more images of a skin lesion,
      wherein the set of images acquired by the multispectral technology (101), termed as first set of images, being acquired by using different illumination spectral bands, each illumination spectral band used providing an image, to detect color and spectral properties of the skin lesion and their spatial distribution,
      wherein the set of images acquired by the 3D technology (102), termed as second set of images, being acquired by using stereoscopy and/or fringe projection techniques to detect three-dimensional morphological properties of the skin lesion and their spatial distribution, and
      wherein the set of images acquired by the OFI technology (103), termed as third set of images, being acquired to detect blood flow changes of the skin lesion and their spatial distribution;

      - analyzing at least two of said first, second or third sets of images and computing a series of indicators and/or parameters for each one of said at least two analyzed sets of images; and
      - combining the at least two series of computed indicators and/or parameters to characterize the skin lesion.

2. The method of claim 1, wherein the imaging platform further comprises, depending on a result of said characterization, the use of a reflectance confocal microscopy optical technology, the method further comprising storing a set of images acquired by the reflectance confocal microscopy optical technology to form an image of a region of the skin located beneath the skin lesion.

3. The method of claim 1, wherein the series of indicators and/or parameters for the first set of images being computed by:

   - comparing, pixel by pixel, the first set of images of the skin lesion and a reference image computing from the first set of images different radiometric magnitudes, providing a reflectance or absorbance image;
   - computing from said reflectance or absorbance image other images whose intensity pixels represent parameters based on color coordinates or color differences of a color representation space or based on other empirically obtained by operating with reflectance/absorbance values of different wavelengths to enhance particular spectral features, and compute from said other images statistical properties thereof, said statistical properties at least including the computing of a histogram of the intensity of the pixels of said other images; and
   - computing different parameters including central moment, energy, entropy and other first or second order statistical descriptors of the computed histogram.

4. The method of claim 1, wherein the series of indicators and/or parameters for the second set of images being computed by:

- combining at least six images of the second set of images of the skin lesion extracting a 3D topography image;
- computing from the extracted 3D topography image statistical properties thereof, said statistical properties at least including the computing of a histogram of a height distribution of the 3D topography image, a histogram of a curvature distribution of the 3D topography image and a histogram of roughness values of the 3D topography image; and
- computing different parameters including central moment, energy, entropy and other first or second order statistical descriptors of the computed histograms.

5. The method of claim 1, wherein the series of indicators and/or parameters for the third set of images being computed by:

   - extracting a false color image from the third set of images;
   - computing from the false color image statistical properties thereof, said statistical properties at least including the computing of a histogram of the intensity of the pixels of the false color image; and
   - computing different parameters including area, shape, central moment, energy, entropy and skewness of the computed histogram.

6. The method of claim 3, wherein the series of indicators and/or parameters computed for the first set of images being further computed by computing average, standard deviation, maximum and minimum values of absorbance, reflectance or color measurements of the skin lesion.

7. The method of claim 4, wherein the series of indicators and/or parameters computed for the second set of images being further computed by computing average, standard deviation, maximum and minimum values related to surface topography of the skin lesion including perimeter, area, shape, height, slope, curvature, roughness and/or volume.

8. The method of claim 5, wherein the series of indicators and/or parameters for the third set of images being further computed by computing average, standard deviation, maximum and minimum values related with the blood flow of the skin lesion.

9. The method of claim 1, wherein said combining comprises comparing the at least two series of computed indicators and/or parameters, providing a combined set of indicators and/or parameters, and further performing a statistical analysis over the combined set of indicators and/or parameters to consider only those indicators and/or parameters of the combined set having a value over a given threshold.

10. The method of claim 1, wherein said different illumination spectral bands comprises at least eight spectral bands, comprised in the visible and/or in the infrared domain.

11. The method of claim 10, comprising obtaining the at least eight multispectral images through an automated and sequential process.

12. A system to characterize a skin lesion, comprising:

   - a memory (201) adapted to store at least three different sets of images, said at least three different sets of images being acquired by an imaging platform (100) comprising three different optical technologies including a multispectral technology (101), a 3D technology (102) and an optical feedback interferometry, OFI, technology (103),

      wherein each set of images being acquired by one of said optical technologies (101, 102, 103), each set of images comprising one or more images of a skin lesion,
      wherein the set of images acquired by the multispectral technology (101), termed as first set of images, being acquired by using different illumination spectral bands, each illumination spectral band used providing an image, to detect color and spectral properties of the skin lesion and their spatial distribution,
      wherein the set of images acquired by the 3D technology (102), termed as second set of images, being acquired by using stereoscopy and/or fringe projection techniques to detect three-dimensional morphological properties of the skin lesion and their spatial distribution, and
      wherein the set of images acquired by the OFI technology (103), termed as third set of images, being acquired to detect blood flow changes of the skin lesion and their spatial distribution; and

- one or more processors (202) being adapted and configured to execute an algorithm to:

analyze at least two of said first, second or third sets of images computing a series of indicators and/or parameters for each one of said at least two analyzed sets of images; and
combine the at least two series of computed indicators and/or parameters to characterize the skin lesion.

13. The system of claim 12, wherein said memory (201) being further adapted to store a set of images acquired by a reflectance confocal microscopy optical technology to detect a region of the skin located beneath the skin lesion.

14. A computer program product comprising program code instructions that when executed in a computer system implement the method of any of claims 1 to 11.

**Fig. 1**

FIRST SET OF IMAGES OF THE
SKIN LESION

SECOND SET OF IMAGES OF
THE SKIN LESION

ANALYSIS OF THE FIRST AND SECOND SET OF
IMAGES TO COMPUTE A SERIES OF INDICATORS
AND/OR PARAMETERS FOR EACH SET

COMBINATION OF THE COMPUTED SERIES OF
INDICATORS AND/OR PARAMETERS TO
CHARACTERIZE THE SKIN LESION

**Fig. 2**

| FIRST SET OF IMAGES OF THE SKIN LESION | SECOND SET OF IMAGES OF THE SKIN LESION | THIRD SET OF IMAGES OF THE SKIN LESION |
|---|---|---|

ANALYSIS OF ALL SETS OF IMAGES TO COMPUTE A SERIES OF INDICATORS AND/OR PARAMETERS FOR EACH SET

COMBINATION OF THE COMPUTED SERIES OF INDICATORS AND/OR PARAMETERS TO CHARACTERIZE THE SKIN LESION

# Fig. 3

Europäisches
Patentamt

European
Patent Office

Office européen
des brevets

# EUROPEAN SEARCH REPORT

Application Number

EP 17 15 2349

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DELPUEYO, X. ET AL.: "Mejora del diagnóstico del cáncer de piel a través de sistemas ópticos", E-MEDIDA. REVISTA ESPAÑOLA DE METROLOGÍA, no. 5, December 2005 (2005-12), XP002772094, * the whole document * | 1-14 | INV. A61B5/00 |
| Y | US 2016/157725 A1 (MUNOZ LUIS DANIEL [US]) 9 June 2016 (2016-06-09) * abstract * * paragraph [0023] - paragraph [0041] * * paragraph [0067] - paragraph [0068] * * paragraph [0084] * * paragraph [0089] * * figures 1-12 * | 1-14 | |
| Y | JULIEN PERCHOUX ET AL: "Current Developments on Optical Feedback Interferometry as an All-Optical Sensor for Biomedical Applications", SENSORS, vol. 16, no. 5, 13 May 2016 (2016-05-13), page 694, XP055376270, DOI: 10.3390/s16050694 * abstract * * page 15 - page 22 * | 1-14 | TECHNICAL FIELDS SEARCHED (IPC) A61B |
| A | US 2015/374309 A1 (FARKAS DANIEL L [US] ET AL) 31 December 2015 (2015-12-31) * paragraph [0023] - paragraph [0028] * * paragraph [0071] * * paragraph [0132] - paragraph [0137] * * figures 1-21 * | 1-14 | |
| A | US 2014/213897 A1 (IFTIMIA NICUSOR V [US] ET AL) 31 July 2014 (2014-07-31) * paragraph [0007] - paragraph [0017] * | 2 | |

The present search report has been drawn up for all claims

| Place of search | Date of completion of the search | Examiner |
|---|---|---|
| Berlin | 14 July 2017 | Abraham, Volkhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 15 2349

This annex lists the patent family members relating to the patent documents cited in the above-mentioned European search report.
The members are as contained in the European Patent Office EDP file on
The European Patent Office is in no way liable for these particulars which are merely given for the purpose of information.

14-07-2017

| Patent document cited in search report | | Publication date | Patent family member(s) | | Publication date |
|---|---|---|---|---|---|
| US 2016157725 | A1 | 09-06-2016 | US 2016157725 A1 | | 09-06-2016 |
| | | | WO 2016094439 A1 | | 16-06-2016 |
| US 2015374309 | A1 | 31-12-2015 | AU 2014212124 A1 | | 10-09-2015 |
| | | | CA 2900138 A1 | | 07-08-2014 |
| | | | CN 105143448 A | | 09-12-2015 |
| | | | EP 2951301 A1 | | 09-12-2015 |
| | | | US 2015374309 A1 | | 31-12-2015 |
| | | | WO 2014121152 A1 | | 07-08-2014 |
| US 2014213897 | A1 | 31-07-2014 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6208749 B1
- EP 1002253 B1
- AU 2009204227 B2
- US 2012123275 A1
- US 8543519 B2

**Non-patent literature cited in the description**

- **BEKINA, A. ; DIEBELE, I. ; RUBINS, U. ; ZAHARANS, J. ; DERJABO, A. ; SPIGULIS, J.** Multispectral assessment of skin malformations using a modified video-microscope. *Latvian Journal of Physics and Technical Sciences,* 2012, vol. 49 (5), 4-8
- **KAPSOKALYVAS, D. ; BRUSCINO, N. ; ALFIERI, D. ; DE GIORGI, V. ; CANNAROZZO, G. ; CICCHI, R. ; MASSI, D. ; PIMPINELLI, N. ; PAVONE, F.S.** Spectral morphological analysis of skin lesions with a polarization multispectral dermoscope. *Optics express,* 2013, vol. 21 (4), 4826-40
- **EMERY J.D. ; HUNTER, J. ; HALL, P.N. ; WATSON, A.J. ; MONCRIEFF, M. ; WALTER, F.M.** Accuracy of SIAscopy for pigmented skin lesions encountered in primary care: development and validation of a new diagnostic algorithm. *BMC Dermatology,* 2010, vol. 10 (9), 1
- **MONHEIT G. ; COGNETTA A. ; FERRIS L. et al.** he performance of MelaFind: a prospective multicenter study. *Archives of dermatology,* 2011, vol. 147 (2), 188
- **DIEBELE I. ; KUZMINA I. ; LIHACHEV A. ; KAPOSTINSH J. ; DERJABO A. ; VALEINE L ; SPIGULIS J.** Clinical evaluation of melanomas and common nevy by spectral imaging. *Biomedical Optics Express,* 2012, vol. 3 (3), 467-472
- **J. EMERY et al.** Accuracy of SIAscopy for pigmented skin lesions encountered in primary care: development and validation of a new diagnostic algorithm. *BMC Dermatology,* 2010, vol. 10 (9